# EUROPEAN PATENT SPECIFICATION

(11) **EP 1 634 085 B1**
(45) Date of publication and mention of the grant of the patent: **08.07.2009**
(21) Application number: 04739331.9
(22) Date of filing: 25.05.2004
(51) Int. Cl.: G01N 33/74

(54) **RELAXIN AS AN INDEPENDENT RISK FACTOR PREDICTING MORTALITY**
RELAXIN, EIN RISIKOFAKTOR ZUR VORHERSAGE DER MORTALITÄT
RELAXINE EN TANT QUE FACTEUR DE RISQUE INDEPENDANT PREDISANT LA MORTALITE

(30) Priority: 30.05.2003 EP 03012374
(43) Date of publication of application: 15.03.2006
(73) Proprietor: Siemens Healthcare Diagnostics GmbH, 65760 Eschborn (DE)
(72) Inventor: STASCH, Johannes-Peter, 42651 Solingen (DE); GEHRMANN, Mathias, 51373 Leverkusen (DE); HOCHER, Berthold, 10098 Berlin (DE)
(74) Representative: Maier, Daniel Oliver
(86) International application number: PCT/EP2004/005599
(87) International publication number: WO 2004/106940

(56) References cited:
- DSCHIETZIG THOMAS ET AL: "The pregnancy hormone relaxin is a player in human heart failure" FASEB JOURNAL, vol. 15, no. 12, October 2001 (2001-10), pages 2187-2195, XP002195177 ISSN: 0892-6638 cited in the application
- YEUN J Y ET AL: "C-Reactive protein predicts all-cause and cardiovascular mortality in hemodialysis patients." AMERICAN JOURNAL OF KIDNEY DISEASES: THE OFFICIAL JOURNAL OF THE NATIONAL KIDNEY FOUNDATION. UNITED STATES MAR 2000, vol. 35, no. 3, March 2000 (2000-03), pages 469-476, XP008024225 ISSN: 1523-6838
- FERNANDEZ-REYES MARIA JOSE ET AL: "Inflammation and malnutrition as predictors of mortality in patients on hemodialysis" JN JOURNAL OF NEPHROLOGY, vol. 15, no. 2, March 2002 (2002-03), pages 136-143, XP002260579 ISSN: 1121-8428

## Description

### Background

About seventy-five years ago, it was shown that injections of serum from pregnant rabbits induced relaxation of the interpubic ligament of female guinea pigs (1). The hormone responsible for this action was named relaxin. Relaxin is a peptide hormone of about 6 kD with homology to the insulin-like growth factor family (1). Corpus luteum, decidua, and placenta secrete this hormone during pregnancy. It was shown that relaxin has many important roles in pregnancy, including softening effects on connective tissue, reducing uterine contractility and control of mammary gland growth and differentiation (1).

Recently, however, it was recognised that relaxin plays also an essential role in the cardiovascular system. Chronic heart failure patients have increased myocardial relaxin gene expression and elevated plasma relaxin concentrations (2).

It is furthermore known that relaxin stimulates atrial natriuretic peptide secretion from isolated perfused rat hearts (3) and also causes a dose-dependent increase in coronary blood flow via a nitric oxide-mediated mechanism (4).

It is furthermore known that, ESKD patients have a substantially reduced life expectancy. The mortality is about 50 % higher as compared to age-matched humans without ESKD. This is mainly due to cardiovascular diseases (8,9).

It is furthermore known that relaxin is a potent vasodilator of small systemic resistance arteries (5). Beside its direct effects on blood vessels and the heart, relaxin is also involved in the regulation of cardiac (6) as well as renal (7) collagen synthesis.

Recently, it was found that cardiac Troponin T predicts mortality in end-stage kidney disease (ESKD) patients (11). Other previously known risk factors predicting reduced life expectancy of ESKD patients are old age, time on dialysis, diabetes, increased C-reactive protein (CrP) levels, high cholesterol levels, and low albumin levels (see Table 1 and 2) (18).

Knowledge of such indicators for reduced life expectancy, e.g., due to cardiovascular diseases, allows to take precautionary measures and to start specialised treatment in order to reduce the risk stemming from such a condition.

Alternative and independent measures to determine or to quantify a patient's reduced life expectancy are even useful in cases where such novel measures are by themselves not better or more accurate than other measures previously known. This is mainly because an independent Recently, it was found that cardiac Troponin T predicts mortality in end-stage kidney disease (ESKD) patients (11). Other previously known risk factors predicting reduced life expectancy of ESKD patients are old age, time on dialysis, diabetes, increased C-reactive protein (CrP) levels, high cholesterol levels, and low albumin levels (see Table 1 and 2).

Knowledge of such indicators for reduced life expectancy, e.g., due to cardiovascular diseases, allows to take precautionary measures and to start specialised treatment in order to reduce the risk stemming from such a condition.

Alternative and independent measures to determine or to quantify a patient's reduced life expectancy are even useful in cases where such novel measures are by themselves not better or more accurate than other measures previously known. This is mainly because an independent measure to determine or to quantify a patient's reduced life expectancy will in any event help to increase the accuracy of the overall analysis, i.e., when information about the novel measure and information about previously known measures are analysed and considered in combination.

### Description of the invention

Starting from the above mentioned state of the art, the problem to be solved by the current invention is the provision of an alternative and independent measure to characterise (or to quantify) a human individual's reduced life expectancy.

The current invention solves this problem by identifying relaxin (e.g., increased relaxin concentrations) as an independent measure to characterise life expectancy of human individuals suffering from ESKD.

245 patients on long-term haemodialysis were followed for 775 days for all-cause mortality and cardiovascular mortality. Blood samples for analysis of relaxin, CrP, Troponin T, and albumin were taken at study entry. Survival was compared by the Kaplan Meier method and Cox regression analysis. Due to the gender-dependency of relaxin secretion, mortality was analysed in female and male ESKD patients separately. 73 patients died during the observation period, 41 of them died due to cardiovascular diseases. Elevated serum relaxin concentrations had a significant impact on all case mortality in men (relative risk: 2.376; 95% confidence interval: 1.162-4.856; p=0.018) and women (relative risk: 2.495; 95% confidence interval: 1.069-5.821; p=0.034). Subgroup analysis of death cases revealed that elevated relaxin is a risk factor for cardiovascular disease related death in male ESKD patients (relative risk: 3.193; 95% confidence interval: 1.157-8.807; p=0.025) but not in female ESKD patients (relative risk: 1.783; 95% confidence interval: 0.467-6.807; p=0.398).

According to the invention, relaxin is an independent risk factor predicting all-case mortality in women and men with ESKD on chronic haemodialysis. The impact of relaxin on cardiovascular mortality is gender-dependent.

Methods of the current invention comprise:
1. A method of characterising the life expectancy of a human individual, comprising the steps of
   (a) determining the level of relaxin in said human individual,
   (b) comparing said level of relaxin with predetermined data, and
   (c) characterising the life expectancy of said human individual based on
   said comparison, wherein elevated levels are associated with decreased life expectancy, and wherein said human individual is an ESKD patient.
2. A method according to count 1, wherein said life expectancy is affected by a cardiovascular disease.
3. A method according to any of counts 1 to 2, wherein said human individual is a male human individual.
4. A method according to any of counts 1 to 3, wherein said predetermined data is previously acquired exclusively from male or female individuals.
5. A method according to any of counts 1 to 4, wherein said comparison is a comparison of said level of relaxin with a cut-off value determined from the receiver operating command (ROC) curve for said predetermined data.
6. A method according to any of counts 1 to 5, wherein the level of relaxin is determined in whole blood, blood serum, blood plasma, and/or urine.
7. A method according to any of counts 1 to 6, wherein the level of relaxin is determined by
   (a) immunoassay,
   (b) homogeneous immunoassay,
   (c) heterogeneous immunoassay,
   (d) competitive immunoassay,
   (e) sandwich immunoassay, and/or
   (f) mass spectrometry.

### Laboratory Methods

Albumin and creatinin were measured by standardised methods on autoanalysers (Hitachi 747, Hitachi 911 and STA, respectively, from Roche Diagnostics GmbH, Mannheim, Germany). C-reactive protein was measured by standardised methods on an autoanalyser (Dimenson RxL (DAD, Behring Vertriebs GmbH, Schwalbach, Germany). Troponin T was measured with an Elecsys System 2010 (Roche Diagnostics GmbH, Mannheim, Germany). All measured parameters are subject to the quality control management and are certified according to the guidelines of the German Society of Clinical Chemistry. Serum Relaxin concentrations were analysed using a commercial ELISA (Immundiagostik GmbH, Bensheim, Germany) as recently described (2).

### Statistics

The ability of all continuous parameters to discriminate between death and survival were determined using the area under the receiver operating command (ROC) curve to calculate the best cut-off values (10). Since relaxin is secreted in a gender-dependent manner (1), relaxin cut-off values were also calculated for women and men separately. Survival was compared by the Kaplan-Meier method. To control for possible confounding, we did a Cox regression analysis for the whole study population as well as for women and men including relaxin a priori and factors known to have an influence on the endpoint death (age, time on dialysis, diabetes, Troponin T, CrP, albumin and cholesterol). All data were analysed using SPSS for Windows, Version 11.0.

### Results

The underlying renal diseases leading to the necessity of initiating haemodialysis are as follows: Sixty-five of the 245 patients (122 women, 123 men) had diabetic nephropathy, 38 patients had hypertensive nephrosclerosis, 30 patients had chronic glomerulonephritis, 28 patients had autosomal dominant polycystic kidney disease, and 20 patients had analgesic nephropathy. The remainder had various rare kidney diseases. In 40 patients, the underlying renal disease was unknown. The patients had a mean age of 63.5±5.8 years reflecting the current elderly dialysis population in Berlin. Body mass index was 25.2±4.6. The mean time on haemodialysis was 5.0±4.8 years. 157 patients had a pre-existing coronary heart disease. 220 had hypertension. No patient was lost to follow-up. Seventy-three patients died; in 41 cases patients died of cardiovascular disease, including 10 with acute myocardial infarction, 15 of sudden cardiac death, and 14 from chronic heart failure. Infectious diseases were the second most common cause of death and involved 23 cases. Sepsis was the by far most common cause of death in this group (18 cases). Five patients died due to cancer. The four remaining death cases were related to accidents (2x), hyperkalimea (1x), and in one case the cause of death was unknown. Analysis of receiver operating command (ROC) curves for relaxin revealed different optimal cut-off values for the whole study population, women and men reflecting the gender-dependency of this hormone also in an elderly population. The best cut for the discrimination between survivors and non-survivors for the whole study population was 32.7 [pg/ml], for women 38.4 [pg/ml], and for men 29.4 [pg/ml], respectively. Using these cut-off values, we could demonstrate that elevated serum relaxin concentrations in patients on chronic haemodialysis without any actual health problem are independent predictors of all-case mortality within the next two years (table 1). A subgroup analysis of death cases related to cardiovascular diseases revealed that relaxin is an especially important predictor of cardiovascular mortality in male ESKD patients (table 2).

### Discussion

In the present study, we demonstrated for the first time that relaxin is an independent predictor of all-case mortality in female and male end-stage kidney disease patients on chronic haemodialysis. A subgroup analysis revealed that relaxin is especially important as predictor of cardiovascular mortality in male ESKD patients. Our prospective study was performed in an elderly cohort with a mean age of 63.5 years. This reflects the current dialysis population in larger cities in Germany especially in Berlin. However, it is important to note in this context that risk factor predicting mortality in our cohort like diabetes, elevated Troponin T and low albumin are similar to those detected in other large prospective studies (11,12,13). Thus, our finding that relaxin is a predictor of mortality is of general impact in ESKD patients.

Based on the data obtained by investigating the effects of exogenous relaxin in rodents, it was suggested that this hormone exerts compensatory effects in the course of chronic heart failure and also myocardial damage after ischemia-reperfusion injury:
i) Relaxin increases coronary artery flow and decreases cardiac malonyldialdehyd (MDA) production after cardiac ischemia-reperfusion injury (14). MDA's are end-products of lipid peroxidation of cell membranes due to oxygen-derived free radicals after for instance ischemia-reperfusion injury (14,15),
ii) Relaxin stimulates synthesis of atrial natriuretic peptide (3),
iii) Relaxin increases collagen matrix degradation (16), and
iv) prevents coronary thrombotic events by up-regulating tissue plasminogen activator (17).

In support of that notion (compensatory effects of relaxin in the course of chronic heart failure and also ischemic myocardial damage, see above) is the observation that plasma relaxin concentrations and also left ventricular relaxin mRNA concentrations are elevated in patient with chronic heart failure (2).

Relaxin seems to be especially important for the cardiovascular mortality risk of male ESKD patients. Although the number of male ESKD patients (123 men) was low, we could demonstrate that relaxin seems to be even more important in this group with respect to cardiovascular mortality than Troponin T (see table 2), a well known risk factor predicting cardiovascular mortality in ESKD patients (11). The gender dependency of relaxin on the cardiac phenotype was also recently seen in relaxin knockout mice (6). Only male relaxin knockout mice develop a cardiac phenotype (increased left ventricular collagen synthesis and impaired left ventricular function). The molecular pathways explaining the gender-dependency of the impact of relaxin on fatal cardiovascular diseases in ESKD patients and the cardiac phenotype in relaxin knockout mice remains to be clarified.

The following references are considered to be related to the subject matter of the invention and have been referenced above:
1. Bani D. Relaxin: A pleiotropic hormone. Gen Pharmac. 1997; 28:13-22
2. Dschietzig T, Richter C, Bartsch C et al., The pregnancy hormone relaxin is a player in human heart failure. FASEB J. 2001; 15(12):2187-95
3. Toth M, Taskinen P, Ruskoaho H. Relaxin stimulates atrial natriuretic peptide secretion in perfused rat heart. J. Endocrinol 1996; 150, 487-495
4. Bani-Sacchi T, Bigazzi M, Bani D et al., Relaxin-induced increased coronary flow through stimulation of nitric oxide production. Br J Pharmacol 1995; 116, 1589-94
5. Fisher C, MacLean M, Morecroft I et al., Is the pregnancy hormone relaxin also a vasodilator peptide secreted by the heart? Circulation. 2002;106(3):292-5
6. Du XJ, Samuel CS, Gao XM et al., Increased myocardial collagen and ventricular diastolic dysfunction in relaxin deficient mice: a gender-specific phenotype. Cardiovasc Res. 2003; 57(2):395-404
7. Garber SL, Mirochnik Y, Brecklin CS et al., Relaxin decreases renal interstitial fibrosis and slows progression of renal disease. Kidney Int. 2001;59(3):876-82
8. Port FK. Morbidity and mortality in dialysis patients. Kidney Int 1994; 46(6):1728-37
9. USRDS: Excerpt from the United States Renal Data System 1999. Annual Data Report. Patients mortality and survival in ESRD. Am J Kidney Dis 1999; 34; S74-S86.
10. Slowinski T, Neumayer HH, Stolze T, Gossing G, Halle H, Hocher B. Endothelin system in normal and hypertensive pregnancy. Clin Sci (Lond). 2002;103; 48:446-449
11. Dierkes J, Domrose U, Westphal S et al., Cardiac troponin T predicts mortality in patients with end-stage renal disease. Circulation 2000; 102(16):1964-9
12. Cano NJ, Roth H, Aparicio M et al., Malnutrition in hemodialysis diabetic patients: evaluation and prognostic influence. Kidney Int. 2002; 62(2):593-601
13. USRDS: Excerpt from the United States Renal Data System 1999. Annual Data Report. Patients mortality and survival in ESRD. Am J Kidney Dis 1999; 34, S74-S86
14. Masini E, Bani D, Bello MG, Bigazzi M, Mannaioni PF, Sacchi TB. Relaxin counteracts myocardial damage induced by ischemia-reperfusion in isolated guinea pig hearts: evidence for an involvement of nitric oxide. Endocrinology. 1997;138(11):4713-20
15. Bani D, Masini E, Bello MG, Bigazzi M, Sacchi TB. Relaxin protects against myocardial injury caused by ischemia and reperfusion in rat heart. Am J Pathol. 1998;152(5):1367-76
16. Unemori, E. N., Pickford, L. B., Salles, A. L., Piercy, C. E., Grove, B. H., Erikson, M. E., Amento, E. P. (1996) Relaxin induces an extracellular matrix-degrading phenotype in human lung fibroblasts in vitro and inhibits lung fibrosis in a murine model in vivo. J Clin Invest 98; 2739-2745
17. Too, C. K., Weiss, T. J., Bryant-Greenwood, G. D. Relaxin stimulates plasminogen activator secretion by rat granulosa cells in vitro. Endocrinology 1982; 111,1424-1426
18. Yeun, J. Y., Levine, R. A., Mantadilak, V., Kaysen, G. A. C- Reactive protein predicts all - cause and cardiovascular mortality in hemodialysis patients. Am J Kidney Ris 2000 ; 35(3), 469-476.

**Table 1: All-case mortality in ESKD patients on haemodialysis**

| | **All Patients** | | | **Women** | | | **Men** | | |
|---|---|---|---|---|---|---|---|---|---|
| | ***Relative Risk*** | ***95% CI*** | ***p-value*** | ***Relative Risk*** | ***95% CI*** | ***p-value*** | ***Relative Risk*** | ***955 CI*** | ***p-value*** |
| **Age (> 69.5 years)** | 1.538 | 0.900-2.628 | 0.115 | 1.470 | 0.623-3.468 | 0.379 | 1.559 | 0.738-3.294 | 0.244 |
| **Time on Dialysis (>5.5 years)** | 2.220 | 1.291-3.818 | 0.004 | 3.464 | 1.485-8.082 | 0.004 | 1.641 | 0.766-3.513 | 0.202 |
| **Diabetes (yes)** | 3.390 | 1.942-5.917 | 0.000 | 6.993 | 2.639-18.519 | 0.000 | 2.381 | 1.133-4.975 | 0.022 |
| **Troponin T (> 0.054 µg/l)** | 2.553 | 1.405-4.641 | 0.002 | 2.964 | 1.262-6.963 | 0.013 | 2.463 | 0.973-6.233 | 0.057 |
| **CrP (> 0.59 mgldl)** | 1.465 | 0.822-2.614 | 0.196 | 2.818 | 1.076-7.381 | 0.035 | 1.047 | 0.499-2.195 | 0.903 |
| **Cholesterol (< 175.5 mg/dl)** | 1.560 | 0.928-2.625 | 0.093 | 1.675 | 0.734-3.817 | 0.221 | 1.669 | 0.822-3.390 | 0.156 |
| **Albumin (< 3.665 g/dl)** | 1.992 | 1.124-3.534 | 0.018 | 1.869 | 0.699-5.000 | 0.213 | 1.767 | 0.814-3.831 | 0.150 |
| **Relaxin (> cut-off)** | 2.137 | 1.263-3.615 | 0.005 | 2.495 | 1.069-5.821 | 0.034 | 2.376 | 1.162-4.856 | 0.018 |

| | | | | | | | | | |
|---|---|---|---|---|---|---|---|---|---|
| All-case mortality in ESKD patients. The cut-off values for each continuous parameter was determined using the ROC curve (see statistics) for this parameter. These analysis revealed different cut-off values for women (38.4 [pg/ml]), men (29.4 [pg/ml]), and the entire study population (32.7 [pg/ml]), reflecting the gender dependency of relaxin. 95% CI: 95% confidence interval. Elevated relaxin concentration at study entry are an independent predictor of all-case mortality in female and male ESKD patients on haemodialysis. | | | | | | | | | |

**Table 2: Cardiovascular mortality in ESKD patients on haemodialysis**

| | **All Patients** | | | **Women** | | | **Men** | | |
|---|---|---|---|---|---|---|---|---|---|
| | ***Relative Risk*** | ***95% CI*** | ***p-value*** | ***Relative Risk*** | ***95% CI*** | ***p-value*** | ***Relative Risk*** | ***95% CI*** | ***p-value*** |
| **Age (> 69.5 years)** | 1.035 | 0.489-2.192 | 0.928 | 0.417 | 0.123-1.394 | 0.155 | 2.582 | 0.893-7.468 | 0.080 |
| **Time on Dialysis** | 2.087 | 0.981-4.439 | 0.056 | 2.133 | 0.627-7.256 | 0.225 | 2.467 | 0.842-7.233 | 0.100 |
| **Diabetes (yes)** | 5.464 | 2.457-12.195 | 0.000 | 5.128 | 1.330-19.608 | 0.018 | 7.042 | 2.252-22.22 | 0.001 |
| **Troponin T (> 0.054 µg/l)** | 2.328 | 1.037-5.229 | 0.041 | 2.509 | 0.731-8.615 | 0.144 | 1.777 | 0.508-6.213 | 0.368 |
| **CrP (> 0.59 mg/dl)** | 1.729 | 0.752-3.979 | 0.198 | 2.182 | 0.525-9.074 | 0.283 | 1.520 | 0.538-4.298 | 0.430 |
| **Cholesterol (< 175.5 mg/dl)** | 1.761 | 0.858-3.610 | 0.123 | 1.802 | 0.531-6.098 | 0.345 | 2.114 | 0.808-5.525 | 0.127 |
| **Albumin (< 3.665 g/dl)** | 1.597 | 0.723-3.534 | 0.247 | 3.058 | 1.468-13.699 | 0.145 | 0.959 | 0.350-2.625 | 0.935 |
| **Relaxin (> cut-off)** | 2.129 | 1.004-4.512 | 0.049 | 1.783 | 0.467-6.807 | 0.398 | 3.193 | 1.157-8.807 | 0.025 |

| | | | | | | | | | |
|---|---|---|---|---|---|---|---|---|---|
| Mortality due to cardiovascular diseases in ESKD patients. The cut-off values for each continuous parameter was determined using the ROC curve (see statistics) for this parameter. These analysis revealed different cut-off values for women (38.4 [pg/ml]), men (29.4 [pg/ml]), and the entire study population (32.7 [pg/ml]), reflecting the gender dependency of relaxin. 95% CI: 95% confidence interval. Elevated relaxin concentration at study entry are an independent predictor of cardiovascular mortality especially in male ESKD patients. | | | | | | | | | |

## Claims

1. Method of characterising the life expectancy of a human individual, comprising the steps of
a) determining the level of relaxin in a sample obtained from said human individual,
b) comparing said level of relaxin with predetermined data, and
c) characterizing the life expectancy of said human individual based on said comparison wherein elevated levels are associated with decreased life expectancy,
wherein said human individual is an end stage kidney disease (ESKD) patient.

2. Method according to claim 1, wherein said life expectancy is affected by a cardiovascular disease.

3. Method according to any of claims 1 to 2, wherein said human individual is a male human individual.

4. Method according to any of claims 1 to 3, wherein said predetermined data is previously acquired exclusively from male or female individuals.

5. Method according to any of claims 1 to 4, wherein said comparison is a comparison of said level of relaxin with a cut-off value determined from the receiver operating command (ROC) curve for said predetermined data.

6. Method according to any of claims 1 to 5, wherein the level of relaxin is determined in whole blood, blood serum, blood plasma, and/ or urine.

7. Method according to any of claims 1 to 6, wherein the level of relaxin is determined by
a) immunoassay,
b) homogeneous immunoassay,
c) heterogeneous immunoassay,
d) competitive immunoassay,
e) sandwich immunoassay, and/ or
f) mass spectrometry.

## Patentansprüche

1. Verfahren zum Charakterisieren der Lebenserwartung eines menschlichen Individuums, welches die folgenden Schritte umfasst:
a) Bestimmen des Relaxinspiegels in einer Probe, die von dem besagten menschlichen Individuum gewonnen wurde,
b) Vergleichen des besagten Relaxinspiegels mit vorbestimmten Daten, und
c) Charakterisieren der Lebenserwartung des besagten menschlichen Individuums anhand des besagten Vergleichs, wobei erhöhte Spiegel mit verringerter Lebenserwartung verknüpft werden,
wobei das besagte menschliche Individuum ein Patient mit einer Nierenerkrankung im Endstadium ist.

2. Verfahren nach Anspruch 1, wobei die besagte Lebenserwartung durch eine Herz-Kreislauf-Erkrankung beeinträchtigt wird.

3. Verfahren nach einem der Ansprüche 1 bis 2, wobei das besagte menschliche Individuum ein männliches menschliches Individuum ist.

4. Verfahren nach einem der Ansprüche 1 bis 3, wobei die besagten vorbestimmten Daten zuvor ausschließlich von männlichen oder weiblichen Individuen erfasst werden.

5. Verfahren nach einem der Ansprüche 1 bis 4, wobei der besagte Vergleich ein Vergleich des besagten Relaxinspiegels mit einem Cut-Off-Wert ist, der aus der Empfänger-Operationscharakteristik- (Receiver Operating Command, ROC-) Kurve für die besagten vorbestimmten Daten bestimmt wird.

6. Verfahren nach einem der Ansprüche 1 bis 5, wobei der Relaxinspiegel in Vollblut, Blutserum, Blutplasma und/oder Urin bestimmt wird.

7. Verfahren nach einem der Ansprüche 1 bis 6, wobei der Relaxinspiegel bestimmt wird durch
a) Immunoassay,
b) homogenen Immunoassay,
c) heterogenen Immunoassay,
d) kompetitiven Immunoassay,
e) Sandwich-Immunoassay und/oder
f) Massenspektrometrie.

## Revendications

1. Procédé de caractérisation de l'espérance de vie d'un individu humain, comprenant les stades dans lesquels
a) on détermine le taux de relaxine dans un échantillon obtenu à partir de l'individu humain,
b) on compare le taux de relaxine à des données déterminées à l'avance, et
c) on caractérise l'espérance de vie de l'individu humain sur la base de cette comparaison, des taux élevés étant associés à une moindre espérance de vie,
dans lequel l'individu humain est un patient souffrant d'une maladie rénale au stade terminal (MRST).

2. Procédé suivant la revendication 1, dans lequel l'espérance de vie est affectée par une maladie cardiovasculaire.

3. Procédé suivant l'une quelconque des revendications 1 à 2, dans lequel l'individu humain est un individu humain mâle.

4. Procédé suivant l'une quelconque des revendications 1 à 3, dans lequel les données déterminées à l'avance sont acquises au préalable exclusivement chez des individus mâles ou femelles.

5. Procédé suivant l'une quelconque des revendications 1 à 4, dans lequel la comparaison est une comparaison du taux de relaxine à une valeur de coupure déterminée à partir de la courbe receiver operating command (ROC) pour ces données déterminées à l'avance.

6. Procédé suivant l'une quelconque des revendications 1 à 5, dans lequel on détermine le taux de relaxine dans du sang complet, du sérum sanguin, du plasma sanguin et/ou de l'urine.

7. Procédé suivant l'une quelconque des revendications 1 à 6, dans lequel on détermine le taux de relaxine par
a) immunodosage,
b) immunodosage homogène,
c) immunodosage hétérogène,
d) immunodosage compétitif,
e) immunodosage sandwich, et/ou
f) spectrométrie de masse.
